# EUROPEAN PATENT SPECIFICATION

(11) **EP 1 235 574 B1**
(45) Date of publication and mention of the grant of the patent: **02.02.2005**
(21) Application number: 00985142.9
(22) Date of filing: 30.11.2000
(51) Int. Cl.: A61K 31/48, A61K 9/20

(54) **METHODS FOR MAKING SUSTAINED-RELEASE PHARMACEUTICAL COMPOSITIONS OF ERGOT ALKALOIDS HAVING IMPROVED BIOAVAILABILITY AND COMPOSITIONS THEREOF**
ARZNEIZUSAMMENSETZUNGEN UND HERSTELLUNGSVERFAHREN VON ERGOTALKALOIDHALTIGEN ARZNEIZUSAMMENSETZUNGEN MIT VERZÖGERTER WIRKSTOFFABGABE UND VERBESSERTER BIOVERFÜGBARKEIT
PROCEDES PERMETTANT LA PREPARATION DE COMPOSITIONS PHARMACEUTIQUES A LIBERATION PROLONGEE D'ALCALOIDES D'ERGOT PRESENTANT UNE BIODISPONIBILITE AMELIOREE ET COMPOSITIONS AINSI OBTENUES

(30) Priority: 03.12.1999 US 454364
(43) Date of publication of application: 04.09.2002
(60) Divisional application: 04105405.7
(73) Proprietor: Polichem S.A., 1526 Luxembourg (LU)
(72) Inventor: MAILLAND, Federico, I-20121 Milano (IT)
(74) Representative: Dragotti, Gianfranco
(86) International application number: PCT/EP2000/012302
(87) International publication number: WO 2001/039742

(56) References cited:
- EP-A- 0 280 613
- EP-A- 0 284 849
- EP-A- 0 875 245
- FR-A- 2 327 764
- GB-A- 2 170 407
- DATABASE WPI Section Ch, Week 198608 Derwent Publications Ltd., London, GB; Class A96, AN 1986-051909 XP002168552 & JP 61 005010 A (TOA EIYO LTD), 10 January 1986 (1986-01-10)

## Description

### Field of the Invention

This invention relates to methods for making sustained-release pharmaceutical compositions of ergot alkaloids, and particularly to methods for making sustained-release pharmaceutical compositions of ergot alkaloids having improved bioavailability.

### Background of the Invention

In general, ergot alkaloids can be classified according to their different chemical structures, for example ergolines, lysergic acid derivatives, ergot peptide alkaloids and dihydrogenated ergot peptide alkaloids. Clinical applications of ergot alkaloids and their derivatives include treatment of Parkinson's disease, migraine headaches, hyperprolactinemia and cerebro-vascular disturbances, just to name a few.

Many ergot alkaloids and their derivatives are known. For example, U.S. Patent No. 3,896,228 to Richardson discusses the use of ergot alkaloids to increase urine volume and urine pH. U.S. Patent No. 3,987,173 to Borredon proposes the use of certain mixtures of vincamine and ergot alkaloids to treat blood circulation disorders. U.S. Patent No. 4,229,451 to Fehr et al. provides ergopeptine derivatives useful as venoconstrictors and venotonics. U.S. Patent No. 4,315,937 to Maclay et al. discusses ergots and their use in treating minimal brain dysfunction. U.S. Patent No. 4,366,145 to Stoopak et al. discusses a soft gelatin capsule with a liquid ergot alkaloid center fill solution. U.S. Patent No. 4,440,722 to Djorjevic et al. provides a medicine containing salts of ergotamine, ergosinine, ergocryptinine, ergocristinine and ergocominine used for treating arterial hypertension, heart insufficiency, heart arrhythmia or cephalalgia. U.S. Patent No. 4,462,983 to Azria et al. proposes the use of certain ergot peptide alkaloids adapted for nasal or pulmonary administration.

The pharmacological actions of ergot alkaloids are varied and complex, but in general appear to result from their actions at adrenergic, dopaminergic and serotoninergic receptors. The spectrum of effects depends on the agent, dosage, species, tissue, and experimental or physiological conditions. In general, ergot alkaloids are characterized by erratic absorption and a high hepatic first pass effect with wide biotransformation. More specifically, gastrointestinal absorption of ergot alkaloids is low, due to the high hepatic first pass, and sometimes erratic. Moreover, the administration of ergot alkaloids can occasionally be associated with adverse events, particularly vascular and cardiac. Drugs, such as ergot alkaloids, that are susceptible to high hepatic clearance may need to be administered in higher doses in order to maintain blood concentrations above the minimum effective concentration for a sufficient amount of time to provide the desired pharmacological effect. However, when conventional drug delivery systems are used, the burst of drug absorption that occurs just after its administration may cause blood concentrations to exceed the minimum toxic concentration. One method of avoiding this deleterious effect is to employ lower dosage levels with more frequent dosing. Frequent dosing is not an ideal solution, however, because of the inconvenience, the increased cost and the increased likelihood that the patient will forget to take the proper number of doses. Another method of keeping drug concentration on a narrow therapeutically active level is to administer the drug using sustained-release drug delivery systems.

Sustained-release drug delivery systems include any drug delivery system that achieves slow release of the drug over an extended period of time. There are two general types of sustained-release systems: controlled-release and prolonged-release. Controlled-release systems maintain constant drug levels in the target tissue or cells. Prolonged-release systems are unable to maintain a constant drug level, but nevertheless prolong the therapeutic blood or tissue level of the drug for an extended period of time.

When designing sustained-release delivery systems many variables may be considered including the route of drug delivery, the type of delivery system, the specific properties of the drug being administered, and the bioavailability of the drug. Sustained-release delivery systems have been proposed for a number of different drugs. For example, U.S. Patent No. 4,389,393 to Schor et al. proposes sustained release therapeutic compositions based on high molecular weight hydroxypropylmethylcellulose. U.S. Patent No. 5,069,911 to Züger proposes a controlled release formulation for oral administration of a 9, 10-dihydro ergot alkaloid, U.S. Patent No. 5,128,142 to Mulligan et al. proposes a controlled release formulation that includes an absorbate of a mixture of a pharmaceutically useful active ingredient and an inactive substance adsorbed on a cross-link polymer.

While these references propose sustained-release delivery systems that may provide slow release of a particular drug over an extended period of time, they fail to provide such a system that also maintains or increases the bioavailability of the administered drug as compared to conventional delivery systems.

### Summary of the Invention

It is therefore an object of the present invention to provide sustained-release drug delivery systems that maintain or increase the bioavailability of the administered drug as compared to conventional delivery systems.

It is another object of the present invention to provide methods of forming such delivery systems.

These and other objects are provided, according to the present invention, by a method of improving bioavailability of ergot derivatives administered using sustained-release delivery systems comprising combining an ergot derivative or mixture thereof with a pharmaceutically acceptable hydrophilic swelling agent or mixture thereof and one or more pharmaceutically acceptable excipients, wherein the ergot derivative has the formula 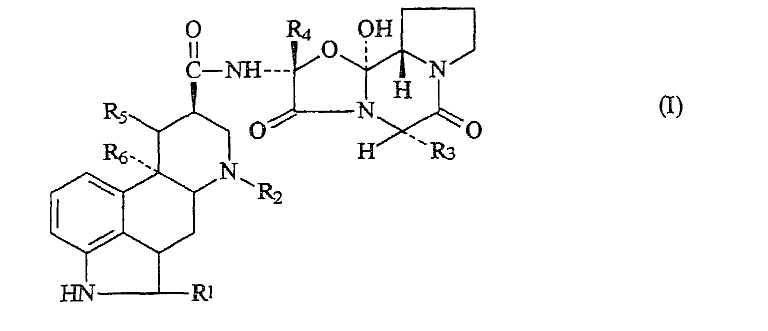 wherein
R₁ is hydrogen or halogen,
R₂ is hydrogen or C₁-C₄ alkyl,
R₃ is isopropyl, sec.-butyl, isobutyl or benzyl,
R₄ is methyl, ethyl, isopropyl, and mixtures thereof, and either
R₅ is hydrogen and
R₆ is hydrogen or methoxy, or
R₅ and R₆ together is an additional bond, and mixtures thereof,
said method being characterized in that the ratio of said ergot derivative to said hydrophilic swelling agent is 1:0.5 to 1:10.

According to the present invention, a pharmaceutical composition comprising α-dihydroergocryptine may also be provided. The composition has a bioavailability at least equal to the bioavailability of the ergot derivative or mixture thereof administered using a conventional delivery system.

Methods and pharmaceutical compositions according to the present invention may therefore provide sustained-release characteristics while improving the bioavailability of ergot derivatives.

### Brief Description of the Drawing

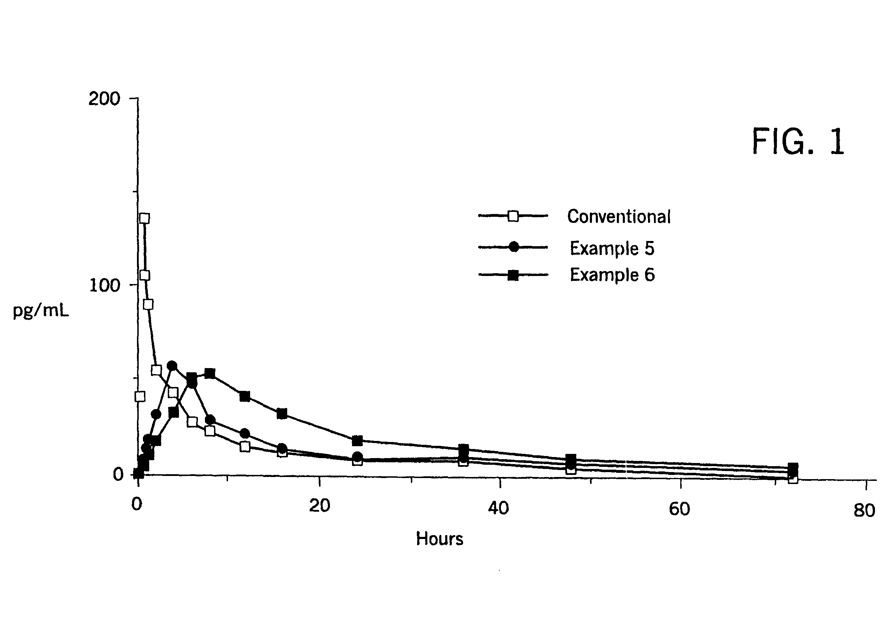

Figure 1 is a graph of plasma concentration of α-dihydroergocryptine versus time after a single oral administration of 10 mg of α-dihydroergocryptine administered in a conventional tablet or in sustained-release tablets formulated according to the present invention as described in examples 5 or 6.

### Detailed Description of Preferred Embodiments

The present invention provides a sustained-release composition of ergot derivatives having an improved bioavailability over conventional compositions. The sustained-release composition of the present invention comprises an ergot derivative or mixture thereof, a pharmaceutically acceptable swelling agent or mixture thereof, and one or more pharmaceutically acceptable excipients.

As used herein, bioavailability is defined as the total amount of drug systemically available over time. Bioavailability may be determined by measuring total systemic drug concentrations over time after administration of a sustained-release composition of the present invention and after administration of a conventional release composition. The improved bioavailability is defined as an increase in the Area Under the Curve (AUC). AUC is the integrated measure of systemic drug concentrations over time in units of mass-time/volume. Following the administration of a drug dose, the AUC from the time of dosing to the time when no drug remains in the body, is a measure of the exposure of the patient to the drug.

Ergot derivatives of the present invention have the formula: 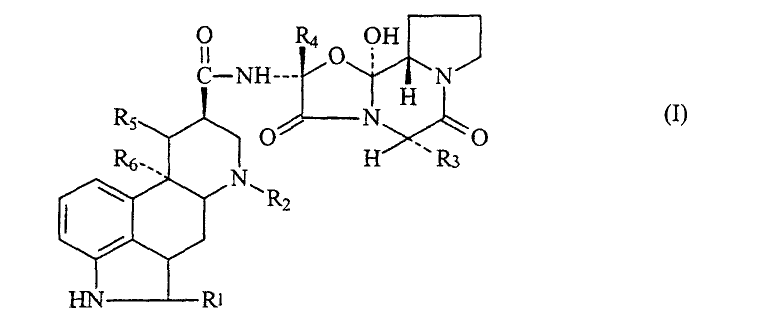 wherein
R₁ is hydrogen or halogen,
R₂ is hydrogen or C₁-C₄ alkyl,
R₃ is isopropyl, sec.-butyl, isobutyl or benzyl,
R₄ is methyl, ethyl, isopropyl, and mixtures thereof, and either
R₅ is hydrogen and
R₆ is hydrogen or methoxy, or
R₅ and R₆ together is an additional bond, and mixtures thereof.
or mixtures thereof.

When R₁ is halogen, it is preferably bromine.

Preferred compounds of Formula I are those in which R₁, R₅ and R₆ are hydrogen, R₂ is methyl, and R₄ is isopropyl or methyl, provided that R₄ is methyl only when R₃ is benzyl.

Particularly preferred compounds in which R₂ is methyl and R₁, R₅ and R₆ are hydrogen are α-dihydroergocryptine (R₄=isopropyl, R₃=isobutyl), β-dihydroergocryptine (R₄=isopropyl, R₅=sec.-butyl), dihydroergocornine (R₃=R₄=isopropyl), dihydroergocristine (R₄=isopropyl, R₃=benzyl) and dihydroergotamine (R₄=methyl, R₃=benzyl), together with their salt forms. The preferred compound in which R₁ is bromine is bromocryptine, R₂=methyl, R₃=isobutyl, R₄=isopropyl and R₅ and R₆ are a second bond. Suitable salt forms are salts of pharmacologically acceptable acids; for example, the methanesulfonate, maleate and tartrate salt forms. The most preferred compound is dihydroergocriptine, usually employed in the form of mesylate. It is indicated for use in the treatment of Parkinson's disease, hyperprolactinemia and migraine. The drug may be administered twice a day at a daily dosage of about 10 to about 60 mg, preferably about 20 to about 40 mg.

Pharmaceutically acceptable swelling agents of the present invention are typically hydrophilic polymers, such as gums, cellulose ethers and protein derived materials. Preferably, these hydrophilic polymers may include hydroxyalkylcelluloses, polyvinyl alcohols, polyoxyethylene glycols and poloxamers. Preferred hydroxyalkylcelluloses include methylcellulose, carboxymethylcellulose, hydroxypropylcellulose and hydroxypropylmethylcellulose.

The most preferred hydrophilic swelling substance is hydroxypropylmethylcellulose. Hydroxypropylmethylcelluloses that may be used in the present invention include Methocel K4M® and Methocel K15M®, both commercially available from Colorcon of West Point, Pennsylvania. Methocel K4M® and Methocel K15M® have a 19-24 weight percent methoxyl content and a 4-12 weight percent hydroxypropyl content. Methocel K4M® in a 2% water solution has a viscosity of 4,000 cps and an average molecular weight of 89,000, while Methocel K15M® in the same conditions has a viscosity of 15,000 cps and an average molecular weight of 124,000.

Formulations of the present invention also contain excipients. In general, excipients include lubricants, suspending agents, binders, diluents, flavorants, colorants, dispersing agents and wetting agents, the use of which will be known to those skilled in the art. Suitable excipients are, in particular, fillers such as sugars, including lactose, sucrose, mannitol, or sorbitol; cellulose preparations such as, for example, microcrystalline cellulose, maize starch, wheat starch, rice starch, potato starch, gelatin, tragacanth gum and/or polyvinylpyrrolidone (PVP); and lubricants such as magnesium stearate.

Formulations of the present invention preferably contain about 5 to about 80 mg of ergot peptide alkaloids. The ratio of ergot peptide alkaloid to swelling substance is preferably from about 1:0.5 to about 1:10, more preferably from about 1:2 to about 1:8. The ratio of dihydroergocriptine to swelling substance is from about 1:0.5 to about 1:5, more preferably from about 1:1 to about 1:4. The ratio of ergot peptide alkaloid to excipients is preferably from about 1:3 to about 1:100, more preferably from about 1:5 to about 1:80 and most preferably from about 1:10 to about 1:50.

Formulations of the present invention provide an increase in bioavailability over other sustained-release formulations. More importantly, formulations of the present invention provide an increase in bioavailability over conventional formulations. The bioavailability of formulations of the present invention is preferably at least about 5%, more preferably at least about 15%, and most preferably at least about 25% higher than the bioavailability of conventional formulations.

The formulations of the present invention may be prepared according to conventional methods by blending together the drug and all the excipients except the lubricant to form a blended powder. The powder is mixed with the lubricant and the resultant powder is pressed to form a tablet.

The following examples are provided to illustrate the present invention, and should not be construed as limiting thereof. In these examples, "mg" means milligram, "ng" means nanogram, "pg" means picogram, "mL" means milliliter, "mm" means millimeter, "°C" means degrees Celsius, "M" means mean, "SD" means standard deviation, "mPa·s" means milliPascal seconds, "PVP" means polyvinylpyrrolidone, "h" means hour and "kp" means kiloponds.

### Examples 1-7 Comparing Release Characteristics of Formulations of the Present Invention with Release Characteristics of Conventional Formulations

### EXAMPLE 1

### α-Dihydroergocryptine 20 mg Sustained-Release Tablets

| *Composition of each tablet* | |
|---|---|
| α-Dihydroergocryptine | 20.0 mg |
| Cellactose® | 203.0 mg¹ |
| Methocel K15M® | 25.0 mg² |
| Syloid 244® | 1.2 mg³ |
| Magnesium stearate | 0.8 mg |

| | |
|---|---|
| Notes: 1. 75% lactose and 25% microcrystalline cellulose, commercially available from Meggle GmbH of Wasserburg, Germany. | |
| 2. Hydroxypropylmethylcellulose USP type 2208; 15,000 mPa s, commercially available from Colorcon of West Point, Pennsylvania. | |
| 3. Silicon dioxide, commercially available from W.R. Grace of Baltimore, Maryland. | |

### Experimental Method

Tablets (250 mg) containing 20 mg (8%) each of α-dihydroergocryptine were prepared with 80% of Cellactose®, as direct compressible excipient, 10% of Methocel K15M® , as swellable controlled release polymer, 1.2% of Syloid 244®, as free flowing agent, and 0.8% of magnesium stearate, as lubricant. The drug and all excipients except the lubricant were geometrically blended manually with a sieve, then mixed with a Turbula mixer for 10 minutes. After adding magnesium stearate, the mixture was blended for another 5 minutes. A rotary 8-station laboratory press with automatic powder feed and capsular tools (12 x 5 mm) was used for compression.

### Tablet Testing

Standard pharmaceutical test methods and equipment were used to determine the following:
Hardness: average of 18.6 kp (Schleuniger 4M)
Friability: 0.081%
Dissolution test, according to USP XXIII, p. 1792, Apparatus 2, 1000 ml H₂0, 50 rotations/min:

| Time (hours) | % Release of α-Dihydroergocryptine (M ± SD) |
|---|---|
| 0.5 | 12.7±0.5 |
| 1 | 23.8±1.4 |
| 2 | 32.6±3.0 |
| 4 | 55.1±6.0 |
| 6 | 72.2±4.5 |
| 8 | 84.7±6.3 |

The conventional tablet formulation of α-dihydroergocryptine 20 mg had the following composition: α-dihydroergocryptine, 20.0 mg; lactose, 148 mg; microcrystalline cellulose, 70 mg; croscarmellose, 6 mg; magnesium stearate, 4 mg; and polyvinylpyrrolidone, 2 mg. The dissolution test was carried out in the same conditions as for the sustained-release formulation of the present invention described in this example and resulted in 96.3 ± 3.6 % release of α-dihydroergocryptine after 0.5 hours.

### EXAMPLE 2

### α-Dihydroergocryptine 20 mg Sustained-Release Tablets

| *Composition of each tablet* | |
|---|---|
| α-Dihydroergocryptine | 20.0 mg |
| Methocel K4M® | 13.2 mg¹ |
| Sodium CMC | 26.8 mg |
| Lactose | 48.0 mg |
| PVP K30 | 6.7 mg |
| Talc | 4.0 mg |
| Magnesium stearate | 1.3 mg |

| | |
|---|---|
| Notes: 1. Hydroxypropylmethylcellulose USP type 2208; 4,000 mPa s, commercially available from Colorcon of West Point, Pennsylvania. | |

### Experimental Method

Tablets (120 mg) containing 20 mg (16.7%) each of α-dihydroergocryptine were prepared and tested according to the methods described hereafter. The formulation was prepared with 40% of lactose, as diluent, 11% of Methocel K4M® and 22.3% of Sodium CMC (medium viscosity), as swellable controlled release polymers, 5.6% of PVP, as binding agent, 3.3% of talc, as anti-sticking agent, and 1.1% of magnesium stearate, as lubricant.

A 10% water solution of PVP was prepared. The drug, the diluent and the polymers were mixed coarsely. The water solution of PVP was added to the powder mix to form a wet mass, which was screened successively through 8 mesh screen.
The wet granulate was dried at 60°C, then screened again through 16 mesh screen. After adding talc and magnesium stearate, the mixture was blended for five minutes in a V mixer. A rotary 8 station laboratory press with automatic powder feed and flat tools, diameter 7 mm, was used for compression.

### Tablet Testing

Standard pharmaceutical test methods and equipment were used for determining the following:
Hardness: average of 11.2 kp (Schleuniger 4M)
Friability: 0.12%
Dissolution test, according to USP XXIII, p. 1792, Apparatus 2, 1000 ml H₂0, 50 rotations/min:

| Time (hours) | % Release of α-Dihydroergocryptine (M ± SD) |
|---|---|
| 0.5 | 12.9 ± 3.6 |
| 1 | 20.8 ± 6.3 |
| 2 | 22.7 ± 6.9 |
| 4 | 35.9 ± 7.7 |
| 6 | 47.6 ± 7.9 |
| 8 | 58.2 ± 10.1 |
| 12 | 68.4 ± 8.0 |

The conventional tablet formulation of α-dihydroergocryptine 20 mg had the same composition as in Example 1. The dissolution test was carried out in the same conditions as for the sustained-release formulation of the present invention described in this example and resulted in 98.1 ± 5.2% release of α-dihydroergocryptine after 0.5 hours.

### EXAMPLE 3

### α-Dihydroergocryptine 40 mg Sustained-Release Tablets

| *Composition of each tablet* | |
|---|---|
| α-Dihydroergocryptine | 40.0 mg |
| Lactose DCL11® | 92.5 mg¹ |
| Avicel PH101® | 76.0 mg² |
| Methocel K4M® | 37.5 mg³ |
| Magnesium stearate | 4.0 mg |

| | |
|---|---|
| Notes: 1. Spray Dried Lactose produced by Meggle GmbH of Wasserburg, Germany. | |
| 2. Microcrystalline cellulose, commercially available from FMC Corporation, Pharmaceuticals Division, of Philadelphia, Pennsylvania. | |
| 3. Hydroxypropylmethylcellulose USP type 2208, 4,000 mPa s, commercially available from Colorcon of West Point, Pennsylvania. | |

### Experimental Method

Tablets (250 mg) containing 40 mg (16%) each of α-dihydroergocryptine were prepared and tested according to the methods described hereafter. The formulation was prepared with 37% of lactose and 30.4% of microcrystalline cellulose, as direct compressible excipients, 15% of Methocel K4M® , as swellable controlled release polymer, and 1.6% of magnesium stearate, as lubricant.

The drug and all excipients except the lubricant were geometrically blended manually with a sieve, then mixed with a Turbula mixer for 10 minutes. After adding magnesium stearate, the mixture was blended for another 5 minutes. A rotary 8 station laboratory press with automatic powder feed and capsular tools (12 x 5 mm) was used for compression.

### Tablet Testing

Standard pharmaceutical test methods and equipment were used to determine the following:
Hardness: average of 14.9 kp (Schleuniger 4M)
Friability: 0.072%
Dissolution test, according to USP XXIII, p. 1792, Apparatus 2, 1000 ml H₂0, 50 rotations/min:

| Time (hours) | % Release of α-Dihydroergocryptine (M±SD) |
|---|---|
| 0.5 | 12.5±0.6 |
| 1 | 22.1±1.4 |
| 2 | 35.9±3.0 |
| 4 | 58.2±4.9 |
| 6 | 74.4±5.4 |
| 8 | 84.0±6.2 |

The conventional tablet formulation of α-dihydroergocryptine 40 mg had the following composition: α-dihydroergocryptine, 40.0 mg; lactose, 128 mg; microcrystalline cellulose, 70 mg; croscarmellose, 6 mg; magnesium stearate, 4 mg; and polyvinylpyrrolidone, 2 mg. The dissolution test was carried out in the same conditions as for the sustained-release formulation of the present invention described in this example and resulted in 93.3 ± 5.0% release of α-dihydroergocryptine after 0.5 hours.

### EXAMPLE 4

### α-Dihydroergocryptine 40 mg Sustained-Release Tablets

| *Composition of each tablet* | |
|---|---|
| α-Dihydroergocryptine | 40.0 mg |
| Lactose DCL11® | 105.0 mg¹ |
| Avicel PH101® | 76.0 mg² |
| Carbopol 934P® | 25.0 mg³ |
| Magnesium stearate | 4.0 mg |

| | |
|---|---|
| Notes: 1. Sprayed dried lactose, commercially available from Meggle GmbH of Wasserburg, Germany. | |
| 2. Microcrystalline cellulose, commercially available from FMC Corporation, Pharmaceuticals Division, of Philadelphia, Pennsylvania. | |
| 3. Carbomer, commercially available from BF Goodrich of Cleveland, Ohio. | |

### Experimental Method

Tablets (250 mg) containing 40 mg (16%) each of α-dihydroergocryptine were prepared and tested according to the methods described hereafter. The formulation was prepared with 42% of lactose and 30.4% of microcrystalline cellulose, as direct compressible excipients, 10% of carbomer, as swellable controlled release polymer, and 1.6% of magnesium stearate, as lubricant.

The drug and all excipients except the lubricant were geometrically blended manually with a sieve, then mixed with a Turbula mixer for 10 minutes. After adding magnesium stearate, the mixture was blended for other 5 minutes. A rotary 8 station laboratory press with automatic powder feed and capsular tools (12 x 5 mm) was used for compression.

### Tablet Testing

Standard pharmaceutical test methods and equipment were used to determine the following:
Hardness: average of 13.2 kp (Schleuniger 4M)
Friability: 0.2%
Dissolution test, according to USP XXIII, p. 1792, Apparatus 2, 1000 ml H₂0, 50 rotations/min:

| Time (hours) | % Release of α-Dihydroergocryptine (M±SD) |
|---|---|
| 0.5 | 5.5±0.9 |
| 1 | 11.2±2.0 |
| 2 | 19.6±6.1 |
| 4 | 30.0±7.1 |
| 6 | 42.5±3.3 |
| 8 | 56.2±4.9 |

The conventional tablet formulation of α-dihydroergocryptine 40 mg had the same composition as in Example 3. The dissolution test was carried out in the same conditions as for the sustained release formulation of the present invention described in this example and resulted in 97.7 ± 6.0% release of α-dihydroergocryptine after 0.5 hours.

### EXAMPLE 5

### α-Dihydroergocryptine 10 mg Sustained-Release Tablets

| *Composition of each tablet* | |
|---|---|
| α-Dihydroergocryptine | 10.0 mg |
| Cellactose® | 184.3 mg¹ |
| Methocel K4M® | 22.0 mg² |
| Methocel K15M® | 9.7 mg³ |
| Sodium CMC | 2.0 mg⁴ |
| Talc | 20.0 mg |
| Magnesium stearate | 2.0 mg |

| | |
|---|---|
| Notes: 1. Composed of 75% lactose and 25% microcrystalline cellulose, commercially available from Meggle GmbH of Wasserburg, Germany. | |
| 2. Hydroxypropylmethylcellulose USP type 2208; 4,000 mPa s. commercially available from Colorcon of West Point, Pennsylvania. | |
| 3. Hydroxypropylmethylcellulose USP type 2208; 15,000 mPa s, commercially available from Colorcon of West Point, Pennsylvania. | |
| 4. Medium viscosity grade. | |

### Experimental Method

Tablets (250 mg) containing 10 mg (4%) each of α-dihydroergocryptine were prepared and tested according to the methods described hereafter. The formulation was prepared with 73.3% of Cellactose®, as direct compressible excipient, 8.8% of Methocel K4M®, 3.9% of Methocel K15M® and 0.8% of sodium CMC as swellable controlled release polymers, 8% of Talc, as anti-sticking agent, and 0.8% of magnesium stearate, as lubricant.

The drug and all excipients except the lubricant were geometrically blended manually with a sieve, then mixed with a Turbula mixer for 10 minutes. After adding magnesium stearate, the mixture was blended for other 5 minutes. A rotary 8 station laboratory press with automatic powder feed and capsular tools (12 x 5 mm) was used for compression.

### Tablet Testing

Standard pharmaceutical test methods and equipment were used to determine the following:
Hardness: average of 19.1 kp (Schleuniger 4M)
Friability: 0.26%
Dissolution test, according to USP XXIII, p. 1792, Apparatus 2, 500 ml 0.01 N HCl, 50 rotations/min:

| Time (hours) | % Release of α-Dihydroergocryptine (M±SD) |
|---|---|
| 0.5 | 14.8±1.0 |
| 1 | 23.6±1.5 |
| 2 | 38.2±1.6 |
| 4 | 60.9±1.1 |
| 6 | 78.5±3.9 |
| 8 | 89.3±4.4 |
| 12 | 98.7±3.8 |

The conventional tablet formulation of α-dihydroergocryptine 10 mg had the following composition: α-dihydroergocryptine, 10 mg; lactose, 158 mg; microcrystalline cellulose, 70 mg; croscarmellose, 6 mg; magnesium stearate, 4 mg; and polyvinylpyrrolidone, 2 mg. The dissolution test was carried out in the same conditions as for the sustained-release formulation of the present invention described in this example and resulted in 96.9 ± 4.8% release of α-dihydroergocryptine after 0.5 hours.

### EXAMPLE 6

### α-Dihydroergocryptine 10 mg Sustained-Release Tablets

| *Composition of each tablet* | |
|---|---|
| α-Dihydroergocryptine | 10.0 mg |
| Cellactose® | 216.0 mg¹ |
| Methocel K4M® | 15.0 mg² |
| Methocel K15M® | 5.0 mg³ |
| Sodium CMC | 2.0 mg⁴ |
| Magnesium stearate | 2.0 mg |

| | |
|---|---|
| Notes: 1. Composed of 75% lactose and 25% microcrystalline cellulose, commercially available from Meggle GmbH of Wasserburg, Germany. | |
| 2. Hydroxypropylmethylcellulose USP type 2208; 4,000 mPa s, commercially available from Colorcon of West Point, Pennsylvania. | |
| 3. Hydroxypropylmethylcellulose USP type 2208; 15,000 mPa s, commercially available from Colorcon of West Point, Pennsylvania. | |
| 4. Medium viscosity grade. | |

### Experimental Method

Tablets (250 mg) containing 10 mg (4%) each of α-dihydroergocryptine were prepared and tested according to the methods described hereafter. The formulation was prepared with 86.4% of Cellactose®, as direct compressible excipient, 6% of Methocel K4M®, 2% of Methocel K15M® and 0.8% of sodium CMC as swellable controlled release polymers, and 0.8% of magnesium stearate, as lubricant.

The drug and all excipients except the lubricant were geometrically blended manually with a sieve, then mixed with a Turbula mixer for 10 minutes. After adding magnesium stearate, the mixture was blended for another 5 minutes. A rotary 8 station laboratory press with automatic powder feed and capsular tools (12 x 5 mm) was used for compression.

### Tablet Testing

Standard pharmaceutical test methods and equipment were used to determine the following:
Hardness: average of 16.1 kp (Schleuniger 4M)
Friability: 0.16%
Dissolution test, according to USP XXIII, p. 1792, Apparatus 2, 500 ml 0.01 N HCl, 50 rotations/min:

| Time (hours) | % Release of α-Dihydroergocryptine (M±SD) |
|---|---|
| 0.5 | 24.7±2.1 |
| 1 | 37.7±2.8 |
| 2 | 58.3±3.2 |
| 4 | 82.6±3.9 |
| 6 | 92.9±3.6 |
| 8 | 97.6±1.8 |
| 12 | 100.00±1.7 |

The conventional tablet formulation of α-dihydroergocryptine 10 mg had the same composition as in Example 5 and the dissolution test carried out in the same conditions as for the sustained release formulation of the present invention described in this example resulted in 93.7 ± 3.1% release of α-dihydroergocryptine after 0.5 hours.

### EXAMPLE 7

### Bromocriptine 5 mg Sustained-Release Tablets

| *Composition of each tablet* | |
|---|---|
| Bromocriptine | 5.0 mg |
| Cellactose® | 189.3 mg¹ |
| Methocel K4M® | 22.0 mg² |
| Methocel K15M® | 9.7 mg³ |
| Sodium CMC | 2.0 mg⁴ |
| Talc | 20.0 mg |
| Magnesium stearate | 2.0 mg |

| | |
|---|---|
| Notes: 1. Composed of 75% lactose and 25% microcrystalline cellulose, commercially available from Meggle GmbH of Wasserburg, Germany. | |
| 2. Hydroxypropylmethylcellulose USP type 2208; 4,000 mPa s, commercially available from Colorcon of West Point, Pennsylvania. | |
| 3. Hydroxypropylmethylcellulose USP type 2208; 15,000 mPa s, commercially available from Colorcon of West Point, Pennsylvania. | |
| 4. Medium viscosity grade. | |

### Experimental Method

Tablets (250 mg) containing 5 mg (2%) each of bromocriptine were prepared and tested according to the methods described hereafter. The formulation was prepared with 75.7% of Cellactose®, as direct compressible excipient, 8.8% of Methocel K4M®, 3.9% of Methocel K15M® and 0.8% of sodium CMC as swellable controlled release polymers, 8% of Talc, as anti-sticking agent, and 0.8% of magnesium stearate, as lubricant.

The drug and all excipients except the lubricant were geometrically blended manually with a sieve, then mixed with a Turbula mixer for 10 minutes. After adding magnesium stearate, the mixture was blended for another 5 minutes. A rotary 8 station laboratory press with automatic powder feed and capsular tools (12 x 5 mm) was used for compression.

### Tablet Testing

Standard pharmaceutical test methods and equipment were used to determine the following:
Hardness: average of 16.4 kp (Schleuniger 4M)
Friability: 0.02%
Dissolution test, according to USP XXIII, p. 1792, Apparatus 2, 500 ml 0.01 N HCl, 50 rotations/min:

| Time (hours) | % Release of α-Dihydroergocryptine (M±SD) |
|---|---|
| 0.5 | 21.8±3.2 |
| 1 | 32.5±4.4 |
| 2 | 47.0±7.4 |
| 4 | 65.6±7.4 |
| 6 | 77.8±8.1 |
| 8 | 90.0±6.9 |

The conventional tablet formulation of bromocriptine 2.5 mg had the following composition: bromocriptine 2.5 mg, lactose 115.5 mg, polyvinylpyrrolidone 4 mg, maleic acid 2 mg, magnesium stearate 1.3 mg, silica collodial 0.35 mg, and maize starch 14 mg. The dissolution test was carried out in the same conditions as for the sustained release formulation of the present invention described in this example and resulted in 96.9 ± 4.8% release of bromocriptine after 0.5 hours.

### Example Comparing Bioavailability of Sustained-Release Formulations of the Present Invention with Bioavailability of Conventional Formulations

### EXAMPLE 8

### Comparative clinical test

The objective of the study was to evaluate in healthy volunteers the pharmacokinetic characteristics and the bioavailability of α-dihydroergocryptine in oral sustained-release tablets according to the present invention as described in Examples 5 and 6 in comparison to a conventional tablet according to the conventional tablet formulation described under Example 5. The study design was an open label, crossover, 3 period design. Twelve male volunteers were randomly assigned to one of three treatment sequences, separated by a one-week wash-out period. The drug was administered orally in the morning in fasted conditions (the fasted conditions were maintained for 4 hours after treatment) in a single dosage of 10 mg. Blood samples were obtained by an indwelling cannula at specific time points up to 72 hours after administration of the drug.

The plasma concentrations throughout the observation period are depicted in Figure 2. The results of the pharmacokinetic analysis carried out on the plasma concentrations are reported in the Table (expressed as mean values).

| | Conventional Release Formulation (from Example 5) | Sustained-Release Formulation of Present Invention (from Example 5) | Sustained-Release Formulation of Present Invention (from Example 6) |
|---|---|---|---|
| Cₘₐₓ (ng/ml) | 147.0 | 65.1 | 58.2 |
| Tₘₐₓ (h) | 1.3 | 4.6 | 8.0 |
| T_{1/2} elim (h) | 14.8 | 27.9 | 42.8 |
| AUCₜₒₜ (ng h/ml) | 865.1 | 1107.0 | 1894.5 |

These data clearly show that both the sustained-release formulations significantly reduce and delay the peak concentration, particularly for the sustained-release formulation described in Example 6. These figures express a slow absorption rate and a dramatic reduction of the burst which usually occurs after administration of a conventional formulation.

A three-fold increase of the elimination half-life is observed for the sustained-release formulation described in Example 6, an index of a prolonged absorption process as compared to the conventional tablet. The bioavailability of sustained release formulations of the present invention, as measured by AUC, is surprisingly higher than the bioavailability obtained with the conventional tablet.

## Claims

1. A sustained-release pharmaceutical composition comprising:
α-dihydroergocryptine; optionally in salt form;
a pharmaceutically acceptable swelling agent or mixture thereof; and
one or more pharmaceutically acceptable excipients;
said composition being **characterized in that** the ratio of α-dihydroergocryptine to said swelling agent from 1:0.5 to 1.5.

2. The composition according to Claim 1, wherein the pharmaceutically acceptable swelling agent is an hydrophilic swelling agent.

3. The composition according to Claim 1, wherein the ratio of α-dihydroergocryptine to said swelling agent is from 1:1 to 1:4.

4. The composition according to Claims 1-3, wherein the hydrophilic swelling agent is selected from the group consisting of methylcellulose, carboxymethylcellulose, hydroxypropylmethylcellulose, polyvinyl alcohols, polyoxyethylene glycols and poloxamers and mixtures thereof.

5. The composition according to Claim 1-3, wherein the one or more pharmaceutically acceptable excipients is selected from the group consisting of lubricants, suspending agents, binders, diluents, flavorants, colorants, dispersing agents and wetting agents.

6. The composition according to Claims 1-5, wherein the ratio of α-dihydroergocryptine to said one or more pharmaceutically acceptable excipients is from 1:10 to 1:50.

7. The composition according to Claim 6, wherein the hydrophilic swelling agent is hydroxypropylmethylcellulose.

8. The composition according to Claims 1-7, wherein 5 to 80 mg of α-dihydroergocryptine is combined.

## Patentansprüche

1. Pharmazeutische Zusammensetzung mit verzögerter Wirkstoffabgabe, umfassend:
α-Dihydroergocryptin beiliebig in Salzform;
ein pharmazeutisch akzeptables Quellungsmittel oder eine Mischung davon; und
ein oder mehrere pharmazeutisch akzeptable(r) wirkstoffträger;
wobei die Zusammensetzung **dadurch gekennzeichnet ist, daß** das Verhältnis von α-Dihydroergocryptin zu dem Quellungsmittel 1:0,5 bis 1:5 beträgt.

2. Zusammensetzung gemäß Anspruch 1, wobei das pharmazeutisch akzeptable Quellungsmittel ein hydrophiles Quellungsmittel ist.

3. Zusammensetzung gemäß Anspruch 1, wobei das Verhältnis von α-Dihydroergocryptin zu dem Quellungsmittel 1:1 bis 1:4 beträgt.

4. Zusammensetzung gemäß den Ansprüchen 1-3, wobei das hydrophile Quellungsmittel aus der Gruppe ausgewählt ist, die aus Methylcellulose, Carboxymethylcellulose, Hydroxypropylmethylcellulose, Polyvinylalkoholen, Polyoxyethylenglycolen und Poloxameren sowie Mischungen davon besteht.

5. Zusammensetzung gemäß den Ansprüchen 1-3, wobei der eine oder die mehreren pharmazeutisch akzeptablen Wirkstoffträger ausgewählt ist (sind) aus der Gruppe, die aus Gleitmitteln, Suspendiermitteln, Bindemitteln, Streckmitteln, Aromastoffen, Färbemitteln, Dispergiermitteln und Benetzungsmitteln besteht.

6. Zusammensetzung gemäß den Ansprüchen 1-5, wobei das Verhältnis von α-Dihydroergocryptin zu dem einen oder den mehreren pharmazeutisch akzeptablen Arzneimittelträger(n) 1:10 bis 1:50 beträgt.

7. Zusammensetzung gemäß Anspruch 6, wobei das hydrophile Quellungsmittel Hydroxypropylmethylcellulose ist.

8. Zusammensetzung gemäß den Ansprüchen 1-7, wobei 5 bis 80 mg α-Dihydroergocryptin kombiniert sind.

## Revendications

1. Composition pharmaceutique à libération prolongée comprenant :
de l'α-dihydroergocryptine facultativement en forme de sel;
un agent de gonflement hydrophile pharmaceutiquement acceptable ou un mélange de ceux-ci ;
et un ou plusieurs excipients pharmaceutiquement acceptables ;
ladite composition étant **caractérisée en ce que** le rapport de l'α-dihydroergocryptine audit agent de gonflement hydrophile est de 1:0,5 à 1:5.

2. Composition selon la revendication 1, dans laquelle l'agent de gonflement pharmaceutiquement acceptable est un agent de gonflement hydrophile.

3. Composition selon la revendication 1, dans laquelle le rapport de l'α-dihydroergocryptine audit agent de gonflement est de 1:1 à 1:4.

4. Composition selon les revendications 1 à 3, dans laquelle l'agent de gonflement hydrophile est choisi dans le groupe comprenant la methylcellulose, la carboxyméthylcellulose. l'hydroxypropylmethylcellulose, les alcools polyvinyliques, les polyoxyéthyléneglycols et les poloxamères et les mélanges de ceux-ci.

5. Composition selon les revendications 1 à 3, dans laquelle les un ou plusieurs excipients pharmaceutiquement acceptables sont choisis dans le groupe comprenant les lubrifiants, les agents de mise en suspension, les liants, les diluants, les aromatisants, les colorants, les agents de mise en dispersion et les agents mouillants.

6. Composition selon les revendications 1 à 5, dans laquelle le rapport de l'α-dihydroergocryptine audit un ou plusieurs excipients pharmaceutiquement acceptables est de 1:10 à 1:50.

7. Composition selon la revendication 6, dans laquelle l'agent de gonflement hydrophile est l'hydroxypropylméthylcellulose.

8. Composition selon les revendications 1 à 7, dans laquelle 5 à 80 mg d'α-dihydroergocryptine sont combinés.
